# EUROPEAN PATENT APPLICATION

(11) **EP 4 563 145 A1**
(43) Date of publication of application: **04.06.2025**
(21) Application number: 23383225.2
(22) Date of filing: 29.11.2023
(51) Int. Cl.: A61K 31/185, A61K 9/00, A61K 38/13, A61K 38/44, A61K 47/00, A61P 27/02

(54) **DIAMINE OXIDASE (DAO) FOR USE IN THE TREATMENT AND/OR PREVENTION OF DRY EYE DISEASE**

(71) Applicant: Dr Healthcare España, S. L., 08017 Barcelona (ES)
(72) Inventor: DUELO RIU, Juan José, 08017 Barcelona (ES); RUIZ LAPUENTE, Carlos Javier, 08006 Barcelona (ES); DE LECEA FLORES DE LEMUS, Carlos, 08023 Barcelona (ES); TINTORÉ GAZULLA, Maria, 08021 Barcelona (ES); CUÑE CASTELLANA, Jordi, 08191 RUBI (ES)
(74) Representative: Ponti & Partners, S.L.P

(57) **Abstract**

The present invention relates to diamine oxidase (DAO) for use in the treatment and/or prevention of dry eye disease.

## Description

### Field of the invention

The present invention relates to diamine oxidase (DAO) for use in the treatment and/or prevention of dry eye disease (DED).

### Background

The dry eye disease (DED) or dry eye syndrome is defined as is a multifactorial disease of the ocular surface characterized by a loss of homeostasis of the tear film, and accompanied by ocular symptoms, in which tear film instability and hyperosmolarity, ocular surface inflammation and damage, and neurosensory abnormalities play etiological roles(See Jennifer P. Craig MCOptom et al., TFOS DEWS II Definition and Classification Report, The Ocular surface, Volume 15, Issue 3, July 2017, Pages 276-283). This disease affects individuals of all ages who present with symptoms and signs suggestive of dry eye disease, such as ocular irritation, redness, mucus discharge, fluctuating vision, and decreased tear meniscus or plugged meibomian glands.

Dry eye disease is generally classified according to a combination of symptoms and signs. Dry eye disease can be classified as mild, moderate, and severe based on both symptoms and signs, but with an emphasis on symptoms over signs. Owing to the nature of dry eye disease, this classification is sometimes imprecise because characteristics at each level overlap. A questionnaire for self-reporting DED symptoms is often used (see for example, https://eyewiki.org/Dry Eye Syndrome Questionnaires). The item 3 in said document relates to OSDI (Ocular Surface Disease Index) which will be used in the examples herein. As mentioned, said OSDI was created in order to quickly assess the symptoms of ocular irritation in dry eye disease and how they affect functioning related to vision. This 12-item questionnaire assesses dry eye symptoms and the effects it has on vision-related function in the past week of the patient's life. The questionnaire has 3 subscales: ocular symptoms, vision-related function, and environmental triggers. Patients rate their responses on a 0 to 4 scale with 0 corresponding to "none of the time" and 4 corresponding to "all of the time." A final score is calculated which ranges from 0 to 100 with scores 0 to 12 representing normal, 13 to 22 representing mild dry eye disease, 23 to 32 representing moderate dry eye disease, and greater than 33 representing severe dry eye disease.

Patients with mild dry eye disease may have symptoms of irritation, itching, soreness, ocular discomfort, burning, or intermittent blurred vision. The diagnosis of dry eye in its mild form is difficult to make because of the inconsistent correlation between reported symptoms and clinical signs. Patients can identify ocular dysesthesia related to contact lens wear or other cause as dryness, even when tear function is normal. More effective relief of patient symptoms can be achieved if the ophthalmologist can differentiate conditions related to dry eye from other causes. Because most dry eye conditions have a chronic course, repeated observation and reporting of symptoms over time will allow clinical diagnosis of dry eye in most cases.

Patients with moderate dry eye disease have increased discomfort and frequency of symptoms, and the negative effect on visual function may become more consistent.

Patients with severe dry eye disease have an increasing frequency of visual symptoms that may become constant as well as potentially disabling. Severe DED also impairs ability of reading or driving and might often disturb normal sleep.

Dry eye disease is also categorized into one of two forms, aqueous tear deficiency and evaporative. These conditions coexist in the majority of the patients with the disease and might vary in their proportion within each individual patient in different seasons.

Patients with dry eye symptoms often have many contributory factors. It is imperative to treat any causative factors that are amenable to treatment. Tear replacement is frequently unsuccessful when used as the sole treatment if additional causative factors are not concomitantly addressed. Patient education is an important aspect of successful management of this condition.

Some of the treatments currently employed against dry eye disease are as follows:
- Omega-3 fatty acid products without ethyl esters have been recommended and widely used in the treatment of dry eye. However, a prospective, multicenter, masked large-scale trial of 3000 mg of omega-3 fatty acids for 12 months did not show any benefit in patient symptoms or signs over placebo. Omega-7 fatty acids have been recently added.
- Topical cyclosporine treatment has long been used in the treatment of dry eye and shown to have clinical benefits. Topical cyclosporine, in some instances, leads to long-term treatment-free remission of patient symptoms and signs.
- Lifitegrast is a lymphocyte function-associated antigen-1 antagonist developed to treat dry eye syndrome (also known as dry eye disease), but the exact mechanism of action of lifitegrast in dry eye is unknown. Topical lifitegrast 5% has been approved by the US Food and Drug

Administration for treatment of dry eye. Published studies show benefit in signs (corneal and conjunctival staining) as well as symptoms (eye dryness score and ocular discomfort) over a period of 3 months. Although the drug seems to be safe over 12 months, long-term efficacy and side effects are unknown. - Other treatments such as oral secretagogues, autologous/allogeneic serum eye drops, therapeutic contact lens, amniotic membrane grafts, surgical or non-surgical punctual occlusion, insuline eye drugs.

Accordingly, the problem to be solved by the present invention is to find an alternative way for treating, improving and the dry eye disease or syndrome. The present invention addresses this problem by employing DAO.

### Summary of the invention

The present invention relates to diamine oxidase (DAO) for use in the treatment or prevention of dry eye disease (DED) or syndrome.

### Detailed description

The present invention relates to diamine oxidase (DAO) for use in the treatment and/or prevention of dry eye disease (DED) or syndrome.

In an embodiment, said prevention is the prevention of recurrence in subjects that have had previous episodes of dry eye disease or syndrome.

"**DAO**" is the abbreviation used to designate the enzyme diamine oxidase responsible for the catalysis of the oxidative deamination of the primary amine group of histamine to give imidazole acetaldehyde. DAO in the present invention may have different origin, e.g. a non-plant origin, plant origin or biotechnological origin. "Non-plant origin" means any DAO that is not obtained from plants but from animal organisms or other non-plant organisms. Thus, this definition includes all DAO isolated from living creatures that are not plants. "Plant origin" means any DAO obtained from plant organisms. "Biotechnological origin" means any DAO prepared from recombinant cell cultures or in non-plant organisms of any type after isolating the DNA for DAO.

"**Dry eye disease**" or "**dry eye syndrome**" or "DED" as defined above make reference to as a group of disorders of the tear film that are due to reduced tear production or tear film instability, associated with ocular discomfort and/or visual symptoms and inflammatory disease od the ocular surface. As mentioned above, "dry eye disease" includes dry eye due to a deficiency in the production of aqueous tears, a loss because of evaporation due to Meibomian gland dysfunction (MGD) or mucodefficient dry eye.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It should be noted that the term "approximately" or "about" applied to the values used earlier and later in this document includes a margin of error of ± 5 %, such as, for example, ± 4 %, ± 3%, ± 2 %, ± 1 %.

As used herein, the term "treat" or "treatment" or "treating" and its cognates refer to an amelioration/improvement of at least one discernible symptom of dry eye disease or dry eye syndrome, or improvement in the self-reported DED questionnaires. In another embodiment, "treat" refers to an amelioration/improvement of at least one measurable physical parameter, not necessarily discernible by the patient, in another embodiment, "treat" or "treatment" or "treating" refer to inhibiting the progression of at least one discernible symptom of dry eye disease or dry eye syndrome, either physically (e.g., stabilization of a discernible symptom), physiologically (e.g., stabilization of a physical parameter), or both. In another embodiment, "treat" or "treatment" or "treating" refer to slowing the progression or reversing the progression of at least one discernible symptom of dry eye disease or dry eye syndrome. As used herein, "prevent" or "prevention" and its cognates refer to delaying the onset or reducing the risk of acquiring or recurring at least one discernible symptom of dry eye disease or dry eye syndrome. The general terms "prevent" or "prevention" also include the prevention of recurrence in subjects that have had previous episodes of DED.

As used herein, the term "preventing and/or treating" includes "preventing and treating" and "preventing or treating".

As used herein the term "supplementation" includes "dietary management", "administration of food supplements, dietary supplements or food for special medical purposes".

In another embodiment, said diamine oxidase (DAO) is combined with at least one another active ingredient. Said at least another active ingredient might be a compound selected from CsA (cyclosporin A), ocular surface surfactant, omega 3 fatty acid, omega 7 fatty acid, a resolvin, an inmmunomodulator, an androgenic hormone and an estrogenic hormone, and even artificial tears with hyaluronic acid, and a combination thereof Preferably, said at least another active ingredient is selected from CsA, an omega 3 fatty acid, an omega 7 fatty acid, a resolvin, and a combination thereof. As said at least another active ingredient it is also possible to have on or more of different types for the same compound, for example one or more ocular surface surfactants, one or more omega 3 fatty acids, one or more omega 7 fatty acids, one or more resolvins, one or more inmmunomodulators, one or more androgenic hormones and one or more estrogenic hormones.

In a preferred embodiment, said diamine oxidase is included in a pharmaceutical composition. In a further preferred embodiment, said pharmaceutical composition further comprises a pharmaceutically acceptable vehicle, adjuvant, diluent or excipient, and optionally at least another active ingredient. Said pharmaceutically acceptable vehicle, adjuvant, diluent or excipient can be any of preserving agents, fillers, disintegrating agents, humidifying agents, emulsifying agents, suspending agents, solvents, dispersion media, coatings, isotonic and absorption delaying agents and the like. The use of such media and agents for pharmaceutical active substances is well known in the art. Except insofar as any conventional media or agent is incompatible with the active ingredient, its use in the therapeutic compositions is contemplated.

In another preferred embodiment, said prevention and/or treatment comprises topically, orally, preferably orally, administering said diamine oxidase or the pharmaceutical composition comprising thereof.

In a preferred embodiment, the DAO or the pharmaceutical composition comprising thereof is administered topically. The dosage form may be selected from spray, ointment, bead suspension, nanoparticles, liposomes and eye drops.

In a more preferred embodiment, the DAO or the pharmaceutical composition comprising thereof is administered orally. The dosage form may be selected from a tablet, capsule, microcapsule, minicapsule, nanocapsule, sachet, liposome, and drink.. In another preferred embodiment, said dosage forms may have gastric protection for protecting the DAO from gastric acidity. The enteric coating layer that coats the various forms rapidly disintegrates or dissolves in a neutral or alkaline medium. The enteric coating layers may contain pharmaceutically acceptable plasticisers to obtain the desired mechanical properties of flexibility and hardness. These plasticisers can be, for example, triacetin, citric acid esters, phthalic acid esters, cetyl alcohol, polyethylene glycols, polysorbates or other plasticisers.

The presence of the gastric protection would depend on the dosage form. For example, for topical administration this gastric protection is not necessary. Accordingly, the present invention encompasses dosage forms with and without gastric protection.

The above mentioned dosage forms may also contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavouring agent such as peppermint, or methyl salicylate. In addition, dosage unit forms can contain various other materials that modify the physical form of the dosage unit, for example, coatings of sugar, shellac, or enteric agents. Other oral dosage forms like syrup or elixir may contain sweetening agents, preservatives, dyes, colourings, and flavourings. In addition, the active compounds may be incorporated into fast dissolve, modified-release or sustained-release preparations and formulations, and wherein such sustained-release formulations are preferably bimodal.

DAO or the compositions according to the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

DAO can also be preferably mixed with pharmaceutically acceptable alkaline substances such as salts of phosphoric acid and sodium, potassium, calcium, magnesium and aluminium, carbonic acid, citric acid or other suitably weak organic and inorganic acids; a co-precipitate of aluminium hydroxide/sodium bicarbonate; substances normally used in anti-acid preparations such as hydroxides of aluminium, calcium and magnesium; magnesium oxide or compound substances such as Al₂O₃.6MgO.CO₂.12H₂O, (Mg₆Al₂(OH)₁₆CO₃.4H₂O, MgO.Al₂O₃.2SiO₂.nH₂O or similar compounds; pH buffering substances such as tris(hydroxymethyl)aminomethane, basic amino acids and their salts or other pharmaceutically acceptable pH buffering substances.

DAO or the compositions according to the present invention may also have a local action or systemic action, i.e. local action is that exerted by the drug "in situ", in the place where it is applied, for example, a revulsive, a local anesthetic, or local action in the gut, and systemic action, on the other hand, is that which occurs at a distance, once the drug that has been absorbed, passes into the blood and is distributed throughout the body. In the context of the present invention, a local action can be achieved, but not limited thereto, with an intracapsular depot system, oral/dental extended release implant or subtenonian release depot system. A systemic action can be achieved, but not limited thereto, with topical omega 3, inmmunomodulators, androgenic and estrogenic hormones.

In another preferred embodiment, said diamine oxidase is included in a functional food or a dietary supplement (for example, refreshment, foodstuffs, genetically-modified fruits, chewing gums, sweets or candy), alone or optionally combined with at least one another active ingredient as mentioned above. In other words, said diamine oxidase is included in food matrices providing them with the enzymatic activity (functional food) and helping in reducing their content in biogenic amines. Diamine oxidase could be incorporated in such food matrices during the process of elaboration (processing aid) or as an ingredient declared in the composition of the finished product. Said diamine oxidase included in a functional food or a dietary supplement can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like. Said functional food or dietary supplement is preferably orally administered.

"Alone" is meant to be as the unique active ingredient but including any acceptable vehicle, adjuvant, diluent or excipient suitable for such functional food or dietary supplement.

In another preferred embodiment, said diamine oxidase or the pharmaceutical composition comprising thereof is a cosmetical or medical device or is included in powder, semi-solid, semi-liquid or liquid form in a delivery system which is a medical device which facilitates the administration of the enzyme. Said diamine oxidase is present alone or optionally combined with at least one another active ingredient as mentioned above. Said diamine oxidase as a cosmetical or medical device or included in the cosmetical or medical device can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like.

The present disclosure also relates to a method of preventing and/or treating dry eye disease according to the embodiments included herein, comprising the step of administering a therapeutically effective amount or dose of DAO according to the embodiments included herein. The terms "therapeutically effective dose" and "therapeutically effective amount" are used to refer to an amount of the subject compound that results in prevention, delay of onset of symptoms, or amelioration of symptoms in the context of the present invention. A therapeutically effective amount, as well as a therapeutically effective frequency of administration, can be determined by methods known in the art and discussed below.

It is noted that any of the embodiments disclosed herein can be taken alone or combined with any other embodiment disclosed herein unless the context specifies otherwise. In other words, for example, a preferred option of a defined feature can be combined with a more or less preferred option of another feature.

The invention will be now illustrated with the following examples, which do not intend to limit its scope.

### EXAMPLES

### Example 1

The present experiment aims to identify the relationship between genetic AOC1 (Amine Oxidase Copper Containing 1) polymorphism also known as genetic DAO deficiency and dry eye disease. Seventy five subjects with moderate to severe LUTS were enrolled in a prospective pilot cohort study to identify prevalence of genetic DAO deficiency, described as at least one SNP affected in the AOC1 gene.

Results: 74.67% of the enrolled patients presented a genetic predisposition to DAO deficiency, known as at least one SNPs with heterozygous mutation.

### Example 2

Seven volunteers have been supplemented with DAO enzyme for a month. After that time a favorable evolution has been observed in their OSDI (ocular surface disease index):

| n | **7** | **7** |
|---|---|---|
| | **Basal OSDI** | **OSDI after supplementation** |
| Average | 38.967 | 21.107 |
| Standard Deviation | 14.104 | 10.443 |
| Student's t test | | *p* = 0.015 |

The OSDI Test (ocular surface disease index) is a simple test created to establish the severity and classification of dry eye disease according to its symptoms. The score allows establishing a classification of its severity:
- normal (0-12 points)
- mild (13-22 points)
- moderate (23-32 points)
- severe (33-100 points)

A score of 100 corresponds to a total disability, whereas a score of 0 corresponds to no disability (a response "At no time" to all answered questions). Therefore, the change with respect to the baseline value of -12.5 corresponds to an improvement in at least one category in half of the answered questions. In our case we can observe a change of -17.86, and a change from a severe to mild OSDI average.

Ref: https://pubmed.ncbi.nlm.nih.gov/20065224/ (Minimal clinically important difference for the ocular surface index)

## Claims

1. Diamine oxidase (DAO) for use in the treatment and/or prevention of dry eye disease.

2. The diamine oxidase for use according to claim 1, wherein said prevention is the prevention of recurrence in subjects that have had previous episodes of dry eye disease.

3. The diamine oxidase for use according to claim 1 or 2, wherein said DAO is combined with at least one another active ingredient.

4. The diamine oxidase for use according to claim 3, wherein said at least one another active ingredient is cyclosporin A, an ocular surface surfactant, an omega 3 fatty acid, an omega 7 fatty acid, a resolvin, an inmmunomodulator, an androgenic hormone, an estrogenic hormone, and artificial tears with hyaluronic acid, and a combination thereof.

5. The diamine oxidase for use according to claim 4, wherein said at least another active ingredient is selected from CsA, an omega 3 fatty acid, an omega 7 fatty acid, a resolvin, and a combination thereof

6. The diamine oxidase for use according to any of the preceding claims, wherein said diamine oxidase is included in a pharmaceutical composition.

7. The diamine oxidase for use according to claim 6, wherein said pharmaceutical composition further comprises a pharmaceutically acceptable vehicle, adjuvant, diluent or excipient, and optionally at least another active ingredient.

8. The diamine oxidase for use according to any of the preceding claims, wherein said prevention or treatment comprises topically or orally, administering of said diamine oxidase or the pharmaceutical composition comprising thereof.

9. The diamine oxidase for use according to claim 8, wherein said prevention or treatment comprises topically administering said diamine oxidase or the pharmaceutical composition comprising thereof.

10. The diamine oxidase for use according to claim 9, wherein said diamine oxidase or the pharmaceutical composition comprising thereof is administered topically in a dosage form selected from spray, ointment, bead suspension, nanoparticles, liposomes and eye drops.

11. The diamine oxidase for use according to claim 8, wherein said prevention or treatment comprises orally administering said diamine oxidase or the pharmaceutical composition comprising thereof.

12. The diamine oxidase for use according to claim 11, wherein said diamine oxidase or the pharmaceutical composition comprising thereof is administered orally in a dosage form selected from a tablet, capsule, microcapsule, minicapsule, nanocapsule, sachet, liposome and drink.

13. The diamine oxidase for use according to any of the claims 1-5, wherein said diamine oxidase is included in a functional food or a dietary supplement.

14. The diamine oxidase for use according to any of the claims 1-5, wherein said diamine oxidase is a cosmetic or a medical device or is included in powder, semi-solid, semi-liquid or liquid form in a delivery system which is a medical device.
